# EUROPEAN PATENT APPLICATION

(11) **EP 3 892 994 A1**
(43) Date of publication of application: **13.10.2021**
(21) Application number: 20168645.8
(22) Date of filing: 08.04.2020
(51) Int. Cl.: G01N 30/34

(54) **PRE-FRACTIONATION FOR MASS SPECTROMETRIC ANALYSIS**

(71) Applicant: PreOmics GmbH, 82152 Planegg/Martinsried (DE)
(72) Inventor: The designation of the inventor has not yet been filed
(74) Representative: Abseher, Roger

(57) **Abstract**

The present invention relates to a method of fractionating a variety of analytes bound to a matrix, said method comprising: (a) eluting said analytes with a plurality of eluents, wherein a first elution step is effected with an elution buffer comprising a first numbered eluent, and a subsequent (n-th) elution step is effected with an elution buffer comprising an n-th numbered eluent, wherein said n-th numbered eluent has a lower dipole moment and/or a higher hydrophobicity than said first numbered eluent.

## Description

The present invention relates to a method of fractionating and thereby separating a variety of analytes bound to a matrix, said method comprising: (a) eluting said analytes with a plurality of eluents, wherein a first elution step is effected with an elution buffer comprising a first numbered eluent, and a subsequent (n-th) elution step is effected with an elution buffer comprising an n-th numbered eluent, wherein said n-th numbered eluent has a lower dipole moment and/or a higher hydrophobicity than said first numbered eluent

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The combination of liquid chromatography (LC) coupled by Electrospray Ionization (ESI) to mass spectrometry (MS) is an art-established procedure to determine the qualitative and quantitative aspects of complex analyte mixtures. Analytes to be analyzed often span a large dynamic range (largest to smallest abundance of analytes) wherein the LC is used to separate these in space and time to facilitate complete measurements on the MS-side. While a single dimension of separation by LC may be sufficient for some analyte samples, highly complex biological samples often cause a significant proportion of the capacity of the mass spectrometer to be absorbed by only a number of abundant analytes. The art-established approach often fails to detect analytes of low abundance which as such are well within the limit of detection, but nevertheless escape detection owing to the presence of the mentioned analytes of large abundance.

In view of these deficiencies, the technical problem underlying the present invention can be seen in the provision of improved means and methods for analysis, especially in the mass spectrometer. This technical problem is solved by the subject-matter of the enclosed claims.

In the first aspect, the present invention provides a method of fractionating a variety of analytes bound to a matrix said method comprising: (a) eluting said analytes with a plurality of eluents, wherein a first elution step is effected with an elution buffer comprising a first numbered eluent, and a subsequent (n-th) elution step is effected with an elution buffer comprising an n-th numbered eluent, wherein said n-th numbered eluent has a lower dipole moment and/or a higher hydrophobicity than said first numbered eluent.

The term "fractionating" has its art-established meaning. In its most general form it refers to separating analytes present in a mixture to a certain extent. Preferably, the term embraces selective enrichment of certain analytes. In the ideal case, each fraction would separate analytes completely without any overlap (assuming perfect separation and/or arbitrarily small fractions). While this is usually not possible, fractionation is nevertheless perceived as a means to improve the results of any downstream analytical method. The term "downstream analytical method" may refer to a single analytic procedure, but also to a series of more than one analytical procedure. Just to give an example, the method of fractionating in accordance with the first aspect may be followed by liquid chromatography (LC). Using analytical terminology, the method of the first aspect provides for fractionating in a first dimension, and liquid chromatography for fractionating in a second dimension. Liquid chromatography in turn may be followed by mass spectrometry (MS), the combination of LC and MS implementing "downstream analytical method". The method of the first aspect is also referred to as "pre-fractionation" herein. Generally, it is physically separated from the mentioned downstream analytical method. For that reason, pre-fractionation in accordance with the first aspect is also called "offline". On the other hand, in many implementations LC and MS are physically coupled and also referred to as "online".

The term "analyte" refers to a molecular species present in the sample. Samples generally comprise a variety of different analytes: For instance, a sample may comprise a human proteome which is made up of thousands of distinct peptides and these distinct peptides again are present in various quantities, e.g., from a single "copy" of each peptide to millions of "copies". The number of analytes is therefore not particularly limited. In case of analysis of LC-MS based bottom-up proteomics, it has to be understood that routinely proteins are proteolytically digested prior to analysis.

The term "matrix" refers to a material which differentially absorbs analytes. The matrix may be provided in one of various well-known forms such as resin, beads and slurry. It can be provided in column format, such as a prepacked column. It may also be provided as cartridges, which is preferred in accordance with the present invention. A cartridge and more specifically solid phase extraction (SPE) cartridges are generally a consumable which is prefilled with a matrix. SPE-cartridges are often advantageous as they offereasy handling without the necessity of specialized equipment. SPE-cartridges can be used in various forms including but not limited to Spin-columns, positive-pressure, negative-pressure or even attached to flow-pumps. They can be multiplexed to either use in low- or high-throughput applications (e.g. 96x in parallel).

Since analytes in a mixture generally differ from each other in terms of one or more properties such as hydrophobicity, charge status and size, most matrix materials (with the exception of a hypothetical totally inert matrix material) will exhibit differential binding to the analytes. Preferred matrix materials are described further below.

The method of the first aspect starts where loading and optionally washing of the matrix has already occurred. As such, the method of the first aspect focuses on elution. Art-established elution protocols may involve harsh conditions, e.g. where unbinding of virtually all analytes is desirable. Alternatively, gradients of varying quantities of the same eluents may be employed. Gradients provide for continuous or stepwise change of the elution conditions which generally provides for separation of analytes.

In accordance with the present invention, elution is performed stepwise. Deviant from the prior art approaches such as gradients, however, the chemicals employed in different elution buffers do not differ from each other in terms of relative amounts of the same eluent constituents; at least this is not the key notion underlying the present invention. The decisive difference is that in the present invention each elution buffer comprises a distinguished chemical eluent which is specific for this elution buffer. This eluent is referred to as "numbered eluent" in order to make it clear that its use is specific for a particular elution step (and optionally also for a particular elution buffer).

This approach has distinct advantages which are apparent from the data presented in the Examples. To explain further and considering samples which are complex peptide mixtures as a preferred example, the individual peptides obtained by a proteolytic digest of a proteome differ from each other with regard to net surface charge and/or hydrophobicity. Using a plurality of numbered eluents which, as required by the first aspect, differ from each other in terms of hydrophobicity and/or dipole moment, the method in accordance with the first aspect directly addresses and accounts for the different net surface charge and/or different hydrophobicity of the analytes. The elution buffer preferably comprises or is a solvent, and more preferably comprises or is an organic solvent.

As such, the key ingredient of a given elution buffer is the given numbered eluent. Having said that, the elution buffer may comprise further constituents which are disclosed further below.

Both dipole moment and hydrophobicity can be expressed using parameters established in the art. The term "dipole moment" as used herein refers to the electric dipole moment. The electric dipole moment is a measure of the separation of positive and negative electrical charges within a system, here a molecule. Molecules of particular relevance in accordance with the invention are the analytes, in particular peptides, and the constituents of the elution buffer, in particular the above recited numbered eluents. The electric dipole moment is a measure of the polarity of the molecule under consideration. Electric dipole moments are measured in Coulomb meters (C · m). An art-established non-SI unit is Debye (D). A Debye is about 3.33564 x 10⁻³⁰ C · m. Encyclopedia such as the Merck Index contain information about the dipole moment of a vast number of chemicals.

Hydrophobicity may be quantitatively expressed as logP value. The logP value is the decadic logarithm of the partition coefficient or distribution coefficient for a given compound in a mixture of two immiscible eluents at equilibrium. For the purpose of defining logP, these eluents are octanol and water. The mentioned partition coefficient is the concentration of the analyte under consideration in octanol, divided by the concentration of said analyte in water. Accordingly, values of logP which are greater than 1 are indicative of a hydrophobic compound, wherein said compound is the more hydrophobic the larger the logP value is. Hydrophilic compounds will have a logP value below 1.

In a preferred embodiment, said analytes are or comprise peptides, proteins and/or polypeptides.

As mentioned above, the recited proteins and polypeptides are preferably of biologic origin. They may be the result of the lysis of cells of the same or of different types and/or from one or more tissue types.

A commonly used enzyme for proteolytic digestion is trypsin. Having said that, there are numbers of art-established alternative enzymes such as Lys-C, Lys-N, Glu-C and Arg-C.

In the simplest design, different elution steps (and optionally also elution buffers) differ from each other only with regard to the numbered eluent, each numbered eluent being specific for a particular elution step (and optionally also elution buffer).

Furthermore, it is envisaged to use a mixture of at least two different eluents (e.g. different solvents or organic solvents) in at least one eluent step (and optionally also in at least one elution buffer). In such a case, said at least two different eluents are preferably entirely absent from the remainder of the eluent steps (and optionally also elution buffers) used in the method of the first aspect. In the alternative, one constituent, two constituents or all constituents of the mixture of eluents may also be used in other elution steps (and optionally also elution buffer).

An example would be the following: a first elution step (and optionally also first elution buffer) could comprise 3-methyl pyridine as first numbered eluent. A second elution step (and optionally also second elution buffer) could comprise 4-methyl pyridine as numbered eluent. Alternatively, to the extent use is made of mixtures of eluents, a first elution step (and optionally also first elution buffer) could comprise 3-methyl pyridine as eluent, and a second elution step (and optionally also elution buffer) could comprise a mixture of 3-methyl pyridine and 4-methyl pyridine as numbered eluent.

It is not excluded that elution steps and elution buffers comprise further constituents and/or further eluents in addition to the mentioned numbered eluents. However, preferably said additional constituents do not vary between eluents. Variation in that context means both chemical nature and relative amounts. In other words, and within the framework of the examples given above, the elution steps and elution buffers may comprise, in addition to the mentioned substituted pyridines, acetonitrile and optionally water. However, in typical implementations, both the presence of acetonitrile and optionally water as well as the relative amounts of these two compounds would not vary among elution steps and elution buffers. As such, the only property which preferably varies between elution steps and the elution buffers is the chemical nature of the numbered eluent (that can also be made of mixtures of eluents, as discussed above).

In a further preferred embodiment, said matrix is selected from mixed phase matrix, strong cation exchange matrix (SCX) or weak cation exchange matrix (WCX) and reverse phase (RP) matrix. As such, use is preferably made of art-established matrices. Particularly preferred are mixed phase matrices. Within the mixed phase matrices, special preference is given to styrene divinyl benzene - reverse phase sulphonate (SDB-RPS) which consists of a partially sulfonated form of styrene divinyl benzene.

In a further preferred embodiment, said fractionating is discontinuous, effected in SPE cartridges and/or not performed on columns.

The term "discontinuous" refers to a procedure where there is an interruption of flow between applying a given elution buffer and a subsequent elution buffer. Discontinuous fractionating or discontinuous elution is generally not performed on columns, but in SPE cartridges.

In a further preferred embodiment, said plurality of eluents is a number selected from 2 to 96, from 2 to 30, from 3 to 8, or is 3.

In a further preferred embodiment, (a) a different elution buffer is used for each elution step; and/or (b) differently numbered eluents are different compounds or different mixtures of compounds. Especially preferred is option (a) in conjunction with the first of the two alternatives recited in (b). Yet, and as noted above, a given numbered eluent may be a mixture of two or more distinct chemical species.

Also envisaged, but less preferred is that (c) the same elution buffer is used at least twice; and/or (d) at least two differently numbered eluents are the same compound or the same mixture of compounds.

Particularly preferred is that elution buffers differ only with regard to numbered eluents and/or the elution buffers do not implement a concentration gradient and/or a pH gradient. In this preferred embodiment, the art-established notion of a gradient, be it a concentration gradient or a pH gradient, is entirely abandoned.

In a further preferred embodiment, numbered eluents are selected from pyridine, piperidine, pyrrolidine, triethylamine, 3-methyl pyridine, 4-methyl pyridine, 2-methyl pyridine, imidazo[1,2-a]pyridine, pyrrole, N-methyl pyrrole, 4-vinyl pyridine, 2-vinyl pyridine, 3,4-dimethyl pyridine, 2,4-dimethyl pyridine, 3,5-dimethyl pyridine, 2,3-dimethyl pyridine, 2,3,5-trimethyl pyridine and 2,4,6-trimethyl pyridine, preference being given to eluents which are aromatic and/or comprise conjugated double bonds.

Also considered is that a given number of eluent is a mixture of two or more such as three, four or five, more preferably two of the above listed preferred eluents. To give an example, a mixture of equal amounts of 3-methyl pyridine and 4-methyl pyridine may be used.

Especially preferred is that said plurality of eluents comprises or is a first numbered eluent, a second numbered eluent and a third numbered eluent being 3-methyl pyridine, 4-methyl pyridine and 2,4,6-trimethyl pyridine, respectively. The order of the first, second and third elution buffers may be changed and the order is preferably the indicated order. In the most preferred implementation of said particularly preferred embodiment, three elution buffers differ from each other only with regard to the respective numbered eluent. Also in said most preferred implementation, the relative amounts of the constituents of the elution buffers do not vary (preferred additional constituents of the elution buffer are given below).

Preferably, each elution buffer comprises or consists of: (a) 1 to 10% (v/v), preferably 5% (v/v) numbered eluent; and (b) 2 to 80% (v/v), preferably 80% (v/v) of a polar aprotic eluent preferably selected from acetonitrile, ethanol and methanol.

To the extent a numbered eluent is implemented as a mixture of two different chemicals, said chemicals being selected from the list given above, preference is given to equal amounts. For example, if a given numbered eluent is implemented as a mixture of 3-methyl pyridine and 4-methyl pyridine, preference is given to 2.5% (v/v) 3-methyl pyridine and 2.5% (v/v) 4-methyl pyridine.

The remainder up to 100% (v/v) is preferably water, preferably at slightly alkaline pH.

In a further preferred embodiment, said step (a) is preceded by (a') removal of excess reagents such as chemical labelling reagents and/or contaminants.

In many settings, the method in accordance with the first aspect is applied to a matrix with analytes bound, wherein the matrix furthermore contains additional compounds bound which, strictly speaking, are not considered to be analytes, for example because they were not present in the original biological sample or are undesired compounds within said sample. Such additional constituents may be excess reagents or contaminants. An example of an excess reagent are the agents used for isobaric labeling in the field of mass spectrometry.

For that purpose, it is preferred that step (a') is effected by an elution step (and optionally also elution buffer) with an upfront eluent with a higher dipole moment than said first numbered eluent and/or with a lower hydrophobicity than said first numbered eluent. In this implementation, the focus is on the removal of excess reagents and/or contaminants which are predominantly polar or hydrophilic in nature.

As noted above, in a preferred embodiment said excess reagent is a labelling reagent such as an isobaric labelling reagent.

In a further preferred embodiment, said upfront eluent is an ester of carbonic acid with a C₂ to C₅ alkanediol. Preferably, said ester is cyclic. More preferably, said upfront eluent preferably is propylene carbonate. Propylene carbonate is a cyclic compound.

In a second aspect, the present invention provides a chromatographic method, said chromatographic method comprising the method of any of the preceding claims, wherein step (a) and, to the extent present, step (a'), are preceded by a step (a") of loading a sample comprising said variety of analytes onto said matrix.

In a third aspect, the present invention provides a combined offline-online method of analyzing analytes, said method comprising the method of any of the preceding claims, followed by (b) liquid chromatography (LC), and optionally (c) online coupled to said LC, mass spectrometry.

The third aspect defines an embedding of the method of the first or the second aspect into a more complex workflow, which more complex workflow yields a mass spectrum in the end. In the context of the third aspect, the art-established online coupling between LC and MS is not abandoned, but augmented by a step of pre-fractionation, said step of pre-fractionation being implemented by the method of the first or second aspect of the present invention. Pre-fractionating is performed offline, while the art-established online coupling of LC and MS is maintained.

In a preferred embodiment, said LC is performed on a RP column and/or the matrix used in said LC and the matrix of the method of the first or second aspect are orthogonal.

Preferably, said preferred embodiment is combined with the particularly preferred implementation of the matrix in accordance with the first aspect which is a mixed phase matrix.

The terms "orthogonal" and "orthogonality" are established in the art of analytics. They refer to a scenario where at least two different analytical methods are applied. In case of perfect orthogonality, the parameter governing the separation in the first analytic procedure exhibits no correlation with the parameter governing the separation during the second analytic procedure. Examples of orthogonal pairs of analytical methods include: SCX-RP, and hydrophilic interaction chromatography (HILIC)-RP.

In a fourth aspect, the present invention provides a kit comprising or consisting of (a) a plurality of numbered eluents, the numbered eluents being ordered by decreasing dipole moment and/or increasing hydrophobicity; and (b) a matrix suitable for purifying analytes, said analytes preferably being peptides.

In a preferred embodiment, (a') said matrix is as defined in relation to the first aspect and/or is provided as SPE cartridges; (b') said plurality of numbered eluents are as defined in relation to the first aspect; and preferably are as defined in relation to the first aspect; and/or (c') said numbered eluents are comprised in ready-to-use elution buffers selected from elution buffers comprising a first numbered eluent, a second numbered eluent and a third numbered eluent being 3-methyl pyridine, 4-methyl pyridine and 2,4,6-trimethyl pyridine, respectively.

In a further preferred embodiment, said kit further comprises or further consists of one or more of the following: (a) a loading buffer, preferably acidified organic solvents; (b) washing buffers, preferably acidified aqueous buffers and/or acidified organic solvents; and (c) a manual with instructions for performing the method of any of the first, second or third aspect of this invention.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The Examples illustrate the invention.

### Example 1

### Materials and Methods

If not otherwise indicated, use is made of the iST procedure (Kulak et. al., Minimal, encapsulated proteomic-sample processing applied to copy-number estimation in eukaryotic cells. Nat Methods 11, 319-324 (2014); incorporated by reference) utilizing the iST buffers from the iST-kit which can be bought from PreOmics GmbH, besides the ELUTE buffer which is replaced by eluents in accordance with the present invention.

Starting material is 0,6 OD₆₀₀ of S. *cerevisiae,* which is equivalent to 100 µg of protein. 50 µl of LYSE buffer was added to the cell pellet followed by heating and shaking for 10 minutes at 95 °C and 1.000 rpm. After that, the lysate was sonicated on a Bioruptor® Pico for ten cycles, each consisting of 30 seconds sonication and 30 seconds without sonication at 4 °C. Then, 50 µl of resuspended DIGEST was added and incubated at 37 °C for 60 minutes while shaking at 500 rpm.

The digest reaction was quenched adding 50 µl of STOP buffer. The resulting suspension was thoroughly mixed and transferred on an iST-cartridge and then centrifuged for one minute at 3.800 rcf. 200 µl of WASH1 was added followed by centrifugation as just described. This was repeated as before using WASH2. The flow-through was discarded and the cartridge was transferred to a fresh collection tube.

For fraction 1, the elution buffer consists typically of a mono-methylated pyridine, preferably 3-methyl pyridine, with a concentration (v/v) of 5% in 80% acetonitrile. 100 µl of this buffer was added to the cartridge, followed by centrifugation at 1.000 rcf for one minute. After that the cartridge was transferred to a new collection tube and the second elution is performed with a mono-methylated pyridine, preferably 4-methyl pyridine. The elution procedure is repeated a third time with 2,4,6-trimethyl pyridine as elution chemical. All elution chemicals had a concentration of 5% (v/v).

The order of elution buffers may be changed without significant impact on the results discussed in Example 2.

All three fractions were transferred into in an Eppendorf Concentrator under vacuum at 45 °C until completely dry. Typically, 10 µl of the LC-LOAD buffer of the iST-kit were added for resuspending the dried peptides prior to LC/MS-measurement.

As a control, a standard iST preparation is performed.

### Example 2

### Results

We could observe at least a 1,7-fold increase in the peptides and proteins identified by MS and fractionation efficiency of 60% or above compared to the standard iST method.

The most preferred order of eluents/numbered eluents is 3-methyl pyridine, 4-methyl pyridine and 2,4,6-trimethyl pyridine for the final elution.

Without wishing to be bound by any theory, it is considered that an increase in hydrophobicity from 3-methyl pyridine to 2,4,6-trimethyl pyridine leads to elution of less hydrophobic peptides first, followed by more hydrophobic ones at the end. This can be checked using GRAVY values. GRAVY values for peptides or proteins are calculated as the sum of hydropathy values (Kyte and Doolittle, A simple method for displaying the hydropathic character of a protein. J. Mol. Biol. 157, 105-132 (1982)) of all amino acids, divided by the number of residues in the sequence. The GRAVY values range from -4,5 for the most hydrophilic amino acid, which is arginine, to 4,5 for the most hydrophobic amino acid, which is isoleucine.

When checking the GRAVY coefficient for the three fractions for our experiments, we find GRAVY values for fraction 1 of -0.642, -0.492 for fraction 2 and -0.28 for fraction three.

## Claims

1. A method of fractionating a variety of analytes bound to a matrix, said method comprising:
(a) eluting said analytes with a plurality of eluents, wherein a first elution step is effected with an elution buffer comprising a first numbered eluent, and a subsequent (n-th) elution step is effected with an elution buffer comprising an n-th numbered eluent,
wherein said n-th numbered eluent has a lower dipole moment and/or a higher hydrophobicity than said first numbered eluent.

2. The method of claim 1, wherein said analytes are or comprise peptides, proteins and/or polypeptides.

3. The method of claim 1 or 2, wherein said matrix is selected from a strong anion exchange matrix (SAX), strong cation exchange matrix (SCX), weak anion exchange matrix (WAX), weak cation exchange matrix (WCX), reverse phase (RP) matrix or preferably a mixed phase matrix with SCX/WCX and RP character.

4. The method of any one of claims 1 to 3, wherein said plurality of eluents is a number selected from 2 to 96, from 3 to 8, or is 3.

5. The method of any one of the preceding claims, wherein
(a) a different elution buffer is used for each elution step; and/or
(b) differently numbered eluents are different compounds or different mixtures of compounds.

6. The method of any one of the preceding claims, wherein
(c) the same elution buffer is used at least twice; and/or
(d) at least two differently numbered eluents are the same compound or the same mixture of compounds.

7. The method of any one of the preceding claims, wherein eluents differ only with regard to numbered eluents and/or the eluents do not implement a concentration gradient and/or a pH gradient.

8. The method of any one of the preceding claims, wherein numbered eluents are selected from pyridine, piperidine, pyrrolidine, triethylamine, 3-methyl pyridine, 4-methyl pyridine, 2-methyl pyridine, imidazo[1,2-a]pyridine, pyrrole, N-methyl pyrrole, 4-vinyl pyridine, 2-vinyl pyridine, 3,4-dimethyl pyridine, 2,4-dimethyl pyridine, 3,5-dimethyl pyridine, 2,3-dimethyl pyridine, 2,3,5-trimethyl pyridine and 2,4,6-trimethyl pyridine, preference being given to eluents which are aromatic and/or comprise conjugated double bonds.

9. The method of any of the preceding claims, wherein each eluent comprises or consists of:
(a) 1 to 10% (v/v), preferably 5% (v/v) numbered eluent; and
(b) 2 to 80% (v/v), preferably 80% (v/v) of a polar aprotic organic solvent preferably selected from acetonitrile, ethanol or methanol.

10. The method of any one of the preceding claims, wherein step (a) is preceded by
(a') removal of excess reagents such as chemical labelling reagents and/or contaminants with specifically selected eluents.

11. The method of claim 10, wherein step (a') is effected by elution with an upfront eluent with a higher dipole moment than said first numbered eluent and/or with a lower hydrophobicity than said first numbered eluent.

12. The method of claim 11, wherein said upfront eluent is an ester of carbonic acid with a C₂ to C₅ alkanediol, said upfront eluent preferably being propylene carbonate.

13. A chromatographic method, said chromatographic method comprising the method of any of the preceding claims, wherein step (a) and, to the extent present, step (a') are preceded by a step
(a") loading a sample comprising said variety of analytes onto said matrix.

14. A kit comprising or consisting of
(a) a plurality of numbered eluents, the numbered eluents being ordered by decreasing dipole moment and/or increasing hydrophobicity; and
(b) a matrix suitable for fractionating analytes, said analytes preferably being peptides, polypeptides or proteins,
preferably wherein
(a') said matrix is as defined in claim 3 and/or is provided as SPE cartridges;
(b') said plurality of numbered eluents are as defined in claim 7; and preferably are as defined in claim 8; and/or
(c') said numbered eluents are comprised in ready-to-use elution buffers selected from elution buffers comprising a first numbered eluent, a second numbered eluent and a third numbered eluent being 3-methyl pyridine, 4-methyl pyridine and 2,4,6-trimethyl pyridine, respectively.

15. The kit of claim 14, further comprising or further consisting of one or more of the following:
(a) a loading buffer, preferably acidified organic solvents;
(b) washing buffers, preferably acidified aqueous buffers and/or acidified organic solvents; and
(c) a manual with instructions for performing the method of any one of claims 1 to 12.
